(19) Europäisches Patentamt
European Patent Office
Office européen des brevets

(11) Veröffentlichungsnummer: **0 013 408**
**B1**

(12) # EUROPÄISCHE PATENTSCHRIFT

(45) Veröffentlichungstag der Patentschrift: **26.05.82**

(21) Anmeldenummer: **79105320.0**

(22) Anmeldetag: **21.12.79**

(51) Int. Cl.³: **C 07 D 213/74,** C 07 D 213/85, C 07 D 401/04, C 07 D 405/12, C 07 D 413/04, C 07 D 417/04, A 61 K 31/44

(54) **4-Aminopyridine, Verfahren zu ihrer Herstellung und diese enthaltende Arzneimittel.**

(30) Priorität: **08.01.79 DE 2900504**

(43) Veröffentlichungstag der Anmeldung: **23.07.80 Patentblatt 80/15**

(45) Bekanntmachung des Hinweises auf die Patenterteilung: **26.05.82 Patentblatt 82/21**

(84) Benannte Vertragsstaaten: **AT BE CH DE FR GB IT LU NL SE**

(56) Entgegenhaltungen: **keine**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt/Main 80 (DE)**

(72) Erfinder: **Greve, Wilfried, Dr., Am Hohen Stein 34, D-6200 Wiesbaden (DE)**
Erfinder: **von Schuh, Heinzgeorg, Dr., Schöne Aussicht 49, D-6200 Wiesbaden (DE)**
Erfinder: **Anagnostopulos, Hirlsto, Kantstrasse 22, D-6204 Taunusstein (DE)**

**EP 0 013 408 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 4-Aminopyridine, Verfahren zu ihrer Herstellung und diese enthaltende Arzneimittel

In der US-Patentschrift 3 547 935 wird über die Herstellung herbicid wirksamer 3-Nitropyridine unter anderem durch Umsetzung von substituierten 4-Chlor-3-nitropyridinen mit Diäthyl- bzw. Di-n-propylamin berichtet. Derartige Verbindungen mit zusätzlichen Alkylsubstituenten in 2- und/oder 6-Stellung werden in dieser Patentschrift aber nicht offenbart.

Überraschend wurde nun gefunden, dass durch Einführung geeigneter primärer und sekundärer Amine in die 4-Stellung von 2,6-Dialkyl-3-nitro (bzw. cyano)-pyridinen Verbindungen mit hervorragenden pharmakologischen Eigenschaften erhalten werden, bei denen die bronchospasmolytische Wirkung im Vordergrund steht. Sie stellen einen neuartigen Strukturtyp unter den bislang bekannten Bronchospasmolytika dar.

Unter den bisher bekannten Bronchospasmolytika nehmen Theophyllinderivate wie Aminophyllin eine bevorzugte Stellung ein (vgl. B. Hellwig, Moderne Arzneimittel, Stuttgart 1980, S. 1274), da sie keine β-sympathomimetischen Wirkungen entfalten und sich deshalb gut für eine langdauernde Behandlung eignen, wie sie bei Asthma bronchiale stets durchgeführt werden muss. Ein gutes Bronchospasmolytikum sollte deshalb ebenso wie Aminophyllin keinen β-sympathomimetischen Einfluss ausüben, ihm aber an Wirkungsstärke und therapeutischer Breite noch überlegen sein. Dieses Ziel konnte durch die erfindungsgemässen Verbindungen erreicht werden. Darüber hinaus eignen sich die erfindungsgemässen Verbindungen als ideale Ausgangsstoffe zum Aufbau weiterer wertvoller Pharmaka.

Die vorliegende Erfindung betrifft folglich substituierte 4-Aminopyridine einschliesslich der Säureadditionssalze, Verfahren zu ihrer Herstellung und deren Verwendung als Arzneimittel.

Gegenstand der Erfindung sind somit mehrfach substituierte 4-Aminopyridine der Formel

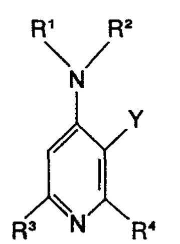

(I)

in der

$R^1$ Wasserstoff oder Alkyl mit bis zu 6 C-Atomen und

$R^2$ a) Alkyl mit bis zu 6 C-Atomen, das bis zu dreifach mit wenigstens einem Rest aus der Gruppe Halogen, Hydroxyl, mit einem Alkohol mit bis zu 3 C-Atomen acetalisiertes Formyl, Alkylthio mit bis zu 3 C-Atomen, Phenylthio, wobei der Phenylring bis zu drei Substituenten der Gruppe Alkyl, Alkoxy, Halogen, Amino und Hydroxy tragen kann, substituiert sein kann, oder

b) Phenylalkyl oder Diphenylalkyl mit bis zu 6 C-Atomen im geraden oder verzweigten Alkylteil, wobei die Phenylringe bis zu dreifach mit wenigstens einem Rest aus der Gruppe Alkyl, Alkoxy, Halogenalkyl mit jeweils bis zu 4 C-Atomen, Halogen, Amino, Hydroxy, der Sulfamoylgruppe und dem Methylendioxyrest substituiert sein können, bedeuten oder

$R^1$ und $R^2$ zusammen mit dem 4-ständigen N-Atom einen fünf- oder sechsgliedrigen heterocyclischen Ring mit bis zu zwei Heteroatomen bilden, der mit Alkyl bis zu 2 C-Atomen substituiert sein kann, wobei das zweite Heteroatom Sauerstoff, Schwefel, der bis zu zwei Sauerstoffatome tragen kann, oder Stickstoff ist, und

$R^3$ und $R^4$ jeweils gleich oder verschieden und Alkyl mit bis zu 9 C-Atomen, wobei einer der beiden Reste auch Wasserstoff sein kann
und

Y eine Nitro- oder Cyanogruppe bedeuten, sowie die physiologisch verträglichen Salze dieser Verbindungen.

Bevorzugt sind solche Verbindungen gemäss Formel I und deren Salze, bei denen $R^3$ und $R^4$ jeweils Alkyl mit bis zu 4 C-Atomen bedeuten und entweder

$R^1$ und $R^2$ zusammen mit dem 4-ständigen N-Atom einen Morpholin- oder Thiomorpholinring bilden oder

$R^1$ Wasserstoff oder Alkyl mit bis zu 4 C-Atomen,

$R^2$ mit Halogen, Alkylthio mit bis zu 3 C-Atomen oder einer gegebenenfalls substituierten Phenylthiogruppe substituiertes Alkyl mit bis zu 4 C-Atomen oder

$R^1$ Wasserstoff,

$R^2$ Benzyl, dessen $CH_2$-Gruppe mit Methyl und dessen Phenylring mit Alkoxy bis zu 2 C-Atomen, Halogen, der Sulfamoylgruppe oder dem Methylendioxy-Rest substituiert sein kann.

Die Verbindungen der Formel I sind neu und besitzen wertvolle pharmakologische, vor allem bronchospasmolytische Eigenschaften.

Gegenstand der Erfindung ist weiterhin ein Verfahren zur Herstellung von 4-Aminopyridinen der Formel I sowie ihrer physiologisch verträglichen Salze, bei dem man

a) eine Verbindung der allgemeinen Formel (II)

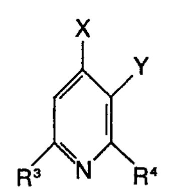

(II)

mit einem Amin der allgemeinen Formel (III)

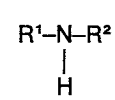

(III)

umsetzt, wobei $R^1$, $R^2$, $R^3$, $R^4$ und Y die oben angegebenen Bedeutungen haben und X ein Halogenatom darstellt, und das erhaltene Produkt isoliert, oder

b) zur Herstellung von Verbindungen der Formel I, in denen $R^2$ ein Halogenatom enthält, eine entsprechende Verbindung der Formel I, in der $R^2$ eine an Alkyl gebundene Hydroxylgruppe trägt, halogeniert, oder

c) zur Herstellung von Verbindungen der Formel I, in denen $R^2$ eine Alkylthio- oder Phenylthiogruppe enthält, die nach Verfahren (b) erhaltene Halogenverbindung mit einem Mercaptan der Formel IV

$$H\text{–}S\text{–}R^5 \qquad (IV)$$

umsetzt, wobei $R^5$ Alkyl mit bis zu 3 Kohlenstoffatomen oder Phenyl bedeutet, das mit den bei $R^2$ (a) genannten Resten substituiert sein kann, oder

d) die nach Verfahren (a) erhaltenen heterocyclischen Thioverbindungen zu Sulfoxyden oder Sulfonen oxidiert,

wobei man die Verbindungen der Formel I entweder als freie Basen isoliert oder mit geeigneten Säuren physiologisch verträgliche Säureadditionssalze bildet.

Für die Herstellung der Säureadditionssalze kommen beispielsweise Halogenwasserstoffsäuren, insbesondere Salzsäure, ferner Schwefel-, Phosphor-, Essig-, Milch-, Malein-, Fumar-, Oxal-, Wein-, Zitronen-, Glukon-, p-Toluolsulfon-, Methansulfon- und Cyclohexylamidosulfonsäure infrage.

Die Ausgangsstoffe der Formeln II bis IV sind zumeist literaturbekannte oder aber nach aus der Literatur bekannten Methoden leicht herstellbar.

Als geeignete Verbindungen der Formel II seien beispielsweise die symmetrischen 2,6-Dialkyl-4-halogen-3-nitropyridine, wie 4-Chlor-2,6-dimethyl-3-nitropyridin [P. Nantka-Namirski, Acta Polon. Pharm. 18, 449 (1961)] und 4-Chlor-2,6-dipropyl-3-nitropyridin, die sich aus Acylessigsäureestern über die 6-Alkyl-3-acyl-2,3-dihydro-2,4-dioxy-pyrane durch Umsetzung mit Ammoniak, anschliessende Nitrierung in 3-Stellung und Halogenierung der 4-Position gewinnen lassen, 2,6-Dialkyl-3-cyano-4-halogenpyridine, wie 4-Chlor-3-cyano-2,6-dimethylpyridin [T. Kato et al., Yakugaku Zasshi 91, 740 (1971)], sowie die unsymmetrischen 2,6-Dialkyl-4-halogen-3-nitropyridine, wie 2-Hexyl-6-methyl- bzw. 2-Methyl-6-hexyl-4-chlor-3-nitropyridin, die man beispielsweise durch Umsetzung entsprechender 3-Alkyl-3-aminoacrylsäureester mit Diketen [T. Kato et al., Yakugaku Zasshi 91, 740 (1971)] oder beliebigen Acylessigsäureestern und anschliessender Verseifung, Decarboxylierung, Nitrierung, Isomerentrennung und Halogenierung aufbauen kann, genannt.

Geeignete Amine gemäss Formel III sind beispielsweise Morpholin, Thiomorpholin, Benzylamin, p-Fluorbenzylamin, p-Sulfamoylbenzylamin, Piperonylamin, p-Methoxybenzylamin, (+)1-Phenyläthylamin und (−)1-Phenyläthylamin, 2-Aminoäthanol, 3-Aminopropanol, 2-Fluoräthylamin, 3-Fluorpropylamin, 2,2,2-Trifluoräthylamin und N-(2-Hydroxyätyhl)-äthylamin.

Geeignete Mercaptane gemäss Formel IV stellen beispielsweise Methyl- und Äthylmercaptan, Thiophenol, 4-Chlor-, 4-Fluor-, 4-Amino-, 4-Dimethylamino-, 4-Methyl-, 4-Methoxy-, 3,4-Dichlor-, 3-Methyl-4-methoxy- und 3-Trifluormethylthiophenol dar.

Die Umsetzung von Verbindungen der Formel II mit den Aminen der Formel III wird zweckmässig in einem Lösungs- oder Verteilungsmittel durchgeführt. Hierfür kommen Alkohole, wie Methanol, Äthanol, Isopropanol, n-Propanol, die verschiedenen Butanole sowie Gemische derselben oder auch deren Mischungen mit Wasser, Äther, wie Tetrahydrofuran und Dioxan, aprotische Lösungsmittel wie Pyridin, Dimethylformamid, Dimethylacetamid, Dimethylsulfoxyd und Hxamethylphosphorsäuretriamid oder Kohlenwasserstoffe wie Benzol, Toluol und Xylol in Frage.

Bei der Kondensationsreaktion arbeitet man vorteilhaft mit der mindestens zweifach molaren Menge des jeweils eingesetzten Amins; auch der Einsatz äquimolarer Mengen beider Reaktionspartner ist möglich, doch empfiehlt sich dann die Zugabe eines säurebindenden Mittels, z.B. eines Alkali- oder Erdalkalihydroxyds oder -carbonats oder auch einer organischen Base, wie Triäthylamin, in mindestens stöchiometrischer Menge. Die Reaktion in erster Stufe wird im allgemeinen bei Temperaturen zwischen 0 °C und dem Siedepunkt des jeweiligen Lösungsmittels, vorzugsweise zwischen 20 und 100 °C durchgeführt, wobei die Reaktionszeit von einigen Minuten bis zu mehreren Stunden reichen kann.

Für die Halogenierung, insbesondere Chlorierung von Verbindungen gemäss Formel I, die in $R^2$ eine an Alkyl gebundene Hydroxylgruppe tragen, werden geeignete Halogenierungs-, insbesondere Chlorierungsmittel, wie Phosphoroxychlorid, Phosphorpentachlorid oder Gemische davon, eine Kombination von Triphenylphosphin mit Tetrachlorkohlenstoff, Thionylchlorid oder Bromid verwendet. Als Lösungsmittel kommen hierfür neben den Halogenierungsmitteln selbst vor allem solche in Frage, die sich unter den Reaktionsbedingungen gegenüber den Reaktionsteilnehmern inert verhalten, z.B. Kohlenwasserstoff, wie Hexan, Benzol und Toluol oder halogenierte Kohlenwasserstoffe, wie Dichlormethan, Chloroform, Tetrachlorkohlenstoff und Chlorbenzol.

Die Halogenierungsreaktionen werden gewöhnlich bei Temperaturen zwischen +10 und 100 °C, vorzugsweise zwischen 20 und 70 °C durchgeführt, wobei die Reaktionszeiten auch hier von einigen Minuten bis zu mehreren Stunden reichen können.

Zur weiteren Umsetzung von Verbindungen gemäss Formel I, die in $R^2$ ein Halogenatom enthalten, mit den Mercaptanen der Formel IV werden vorwiegend Wasser, Alkohole wie Methanol, Äthanol, Propanol, Isopropanol oder deren Mischungen mit Wasser, Dimethylformamid, Dimethylacetamid, Dimethylsulfoxyd, Hexamethylphosphorsäuretriamid oder Pyridin als Lösungsmittel verwendet. Diese Umsetzung mit den Mercaptanen wird vorteilhaft in Gegenwart eines säurebindenden Mittels, wie eines Alkali- oder Erdalkalihydroxyds, zweckmässig bei Temperaturen

zwischen 0 und 150, vorzugsweise zwischen 50 und 100 °C durchgeführt.

Für die Oxydation von Verbindungen der Formel I mit schwefelhaltiger $NR^1R^2$-Gruppe zu Sulfoxyden bzw. Sulfonen eignen sich z.B. Oxydationsmittel wie Natriummetaperjodat, Salpetersäure oder unter Umständen auch elementares Chlor, wobei das Oxydationsmittel selbst oder Wasser als Lösungsmittel dienen kann und die Reaktionstemperaturen im allgemeinen zwischen −20 und 100, vorzugsweise zwischen 0 und 70 °C liegen.

Als Alkylreste für $R^1$, $R^2$, $R^3$ und $R^4$ kommen jeweils z.B. Methyl, Äthyl, Propyl, Isopropyl, n-, iso- oder tert.-Butyl, geradkettiges oder verzweigtes Pentyl oder Hexyl, als $R^3$ und $R^4$ ausserdem noch geradkettiges oder verzweigtes Heptyl, Octyl oder Nonyl, z.B. also der Diisobutyl- oder Triisopropylrest in Betracht. Die heterocyclischen Reste, die zusammen $R^1$ und $R^2$ bilden können, sind z.B. gesättigt, wie der Morpholin- und der Thiomorpholinring, ferner der Pyrrolidin-, der Piperidinring und der Ring der Formel

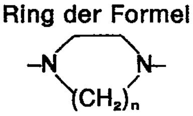

worin n 1 oder 2 ist, oder ungesättigt, wie der 4-H-1,4 4-Thi-azin-Ring.

In allen Fällen kommen als Halogen z.B. Chlor, Brom und Fluor in Betracht, wobei X in der Formel II bevorzugt Chlor oder Brom ist und aliphatisch gebundenes Halogen bevorzugt Fluor oder Chlor ist.

Die neuen 4-Aminopyridine der Formel I und ihre physiologisch verträglichen Salze können aufgrund ihrer pharmakologischen Eigenschaften als Arzneimittel, insbesondere als solche zur Behandlung von Erkrankungen der Atemwege Verwendung finden, wobei man sie entweder allein, z.B. in Form von Mikrokapseln, oder in Kombination mit geeigneten Trägerstoffen verabreicht.

Gegenstand der Erfindung sind somit auch Arzneimittel, die mindestens eine Verbindung der Formel I, gegebenenfalls in Form eines ihrer physiologisch verträglichen Säureadditionssalze, als Wirkstoff enthalten. Die Präparate können oral und parenteral verabreicht werden. Geeignete feste oder flüssige galenische Zubereitungen sind beispielsweise Granulate, Pulver, Tabletten, Dragees, Kapseln, Zäpfchen, Sirupe, Emulsionen, Suspensionen, Tropfen oder injizierbare Lösungen sowie Präparate mit protrahierter Wirkstoff-Freigabe. Als häufig verwendete Trägermittel seien z.B. Magensiumcarbonat, verschiedene Zukker, Stärke, Cellulosederivate, Gelatine, tierische und pflanzliche Öle, Polyäthylenglykole und Lösungsmittel genannt.

Die erforderliche Dosierung bei der Behandlung eines an bronchialen Obstruktionen leidenden erwachsenen Patienten beträgt bei oraler Verabreichung im allgemeinen 30–150 mg pro Tag, vorzugsweise 40–100 mg pro Tag. Bei intravenöser Applikation werden im allgemeinen 10–50 mg pro Tag, vorzugsweise 15–30 mg pro Tag gegeben.

Arzneimittel mit einem Gehalt an Verbindungen der Formel I können auch zusammen mit anderen geeigneten Wirkstoffen, beispielsweise Antiallergika, Antitussiva, Sekretolytika, Sedativa, periphere Vasotherapeutika, Antihistaminika und auch andere Bronchospasmolytika, wie $\beta_2$-Sympathomimetika oder Parasympatholytika formuliert werden.

Beispiele

Die Struktur der nachstehend beschriebenen Verbindungen wurde durch Elementaranalyse und $^1$H-NMR-Spektren bewiesen.

1. 4-(2,6-Dimethyl-3-nitro-4-pyridyl)-thiomorpholinhydrochlorid

Man löst 37,4 g (0,2 Mol) 4-Chlor-2,6-Dimethyl-3-nitropyridin, 26 g (0,25 Mol) Thiomorpholin und 75 ml (0,5 Mol) Triäthylamin in 100 ml Isopropanol und erhitzt 10 Stunden unter Rückfluss. Nach beendeter Reaktion wird das Lösungsmittel unter vermindertem Druck abgezogen. Der Rückstand wird in Chloroform aufgenommen und dreimal mit Wasser ausgeschüttelt. Die Chloroformphase wird getrocknet und eingeengt und zur Überführung ins Hydrochlorid in Äthanol gelöst und mit äthanolischer Salzsäure versetzt. Der Niederschlag wird abgesaugt und aus Methanol umkristallisiert. Ausbeute 44,8 g (77,3% der Theorie); Schmelzpunkt 240 °C (unter Zersetzung) $C_{11}H_{16}$ Cl $N_3$ $O_2$ S (MG = 289,79)

Analyse:

Berechnet:

C 45,59 H 5,57 Cl 12,23 N 14,50 S 11,06%

Gefunden:

C 45,47 H 5,47 Cl 12,16 N 14,49 S 11,16%

2. 4-(2,6-Dimethyl-3-nitro-4-pyridyl)-thiomorpholin-S.S-dioxid-hydrochlorid

Zu 50 ml eisgekühlter, rauchender Salpetersäure (d = 1,5) gibt man portionsweise 5 g (0,02 Mol) 4-(2,6-Dimethyl-3-nitro-4-pyridyl)-thiomorpholin aus Beispiel 1. Man lässt allmählich auf Raumtemperatur kommen und erhitzt anschliessend 6 Stunden auf 70 °C. Nach dem Abkühlen wird vorsichtig mit Wasser verdünnt und unter Eiskühlung mit Natronlauge neutralisiert. Die neutrale, wässrige Lösung wird eingeengt, erneut mit Wasser versetzt und der unlösliche Anteil abfiltriert, aus Methanol umkristallisiert und mit äthanolischer Salzsäure ins Hydrochlorid überführt. Ausbeute: 2 g (31% der Theorie), Schmelzpunkt 255 °C (unter Zersetzung)

$C_{11}H_{16}$ Cl $N_3$ $O_4$ S (MG = 321.78)

Analyse:

Berechnet:

C 41,06 H 5,01 Cl 11,02 N 13,06 S 9,96%

Gefunden:

C 40,67 H 4,97 Cl 11,21 N 12,72 S 9,45%

3. 4-[N-(2-Hydroxyäthyl)]-äthylamino-2,6-dimethyl-3-nitropyridin-hydrochlorid

1,86 g (0,01 Mol) 4-Chlor-2,6-dimethyl-3-nitropyridin, 1,78 g (0,02 Mol) 2-(Äthylamino)-äthanol und 1 g (0,01 Mol) Triäthylamin werden in 10 ml Isopropanol gelöst und 10 Stunden zum Sieden erhitzt. Anschliessend wird eingeengt, mit Wasser aufgenommen und zweimal mit Chloroform ausgeschüttelt. Die Chloroformphase wird getrocknet und eingeengt, die erhaltene Base in Äthanol gelöst und mit äthanolischer Salzsäure ins Hydrochlorid überführt.

Ausbeute: 2,6 g (94,5% der Theorie), Schmelzpunkt 116–118 °C

$C_{11}H_{18}$ Cl $N_3$ $O_3$ (MG = 275,74)

Analyse

Berechnet:

C 47,92 H 6,58 Cl 12,86 N 15,24%

Gefunden:

C 47,89 H 6,68 Cl 13,01 N 15,03%

4. 4-[N-(2-Chloräthyl)]-äthylamino-2,6-dimethyl-3-nitro-pyridin-hydrochlorid

Zu einer Lösung von 2 g (0,0084 Mol) 4-[N-(2-Hydroxyäthyl)]-äthyl-amino-2,6-dimethyl-3-nitropyridin aus Beispiel 3 in 15 ml Chloroform werden 5 ml Thionylchlorid zugetropft und anschliessend 1 Stunde auf 70 °C erwärmt. Nach dem Abkühlen wird mit Wasser versetzt, mit Natriumbicarbonat neutralisiert und mit Chloroform ausgeschüttelt. Die Chloroformphase wird mit Natriumsulfat getrocknet und eingeengt. Das erhaltene Öl wird mit äthanolischer Salzsäure ins Hydrochlorid überführt.

Ausbeute: 1,8 g (73,2% der Theorie), Schmelzpunkt 175–179 °C

$C_{11}$ $H_{17}$ $Cl_2$ $N_3$ $O_2$ (MG = 294,18)

Analyse

Berechnet:

C 44,91 H 5,83 Cl 24,10 N 14,28%

Gefunden:

C 45,03 H 5,94 Cl 24,28 N 14,37%

5. 4-[N-(2-Methylthioäthyl)]-äthylamino-2,6-dimethyl-3-nitropyridin-hydrochlorid

0,48 g (0,012 Mol) Natriumhydroxyd werden in 2 ml Wasser gelöst und mit 10 ml Äthanol verdünnt; unter guter Kühlung mit einer Kältemischung werden zuerst 0,58 g (0,012 Mol) Methylmercaptan zugegeben und anschliessend 2,57 g (0,01 Mol) 4-[N-(2-Chloräthyl)]-äthylamino-2,6-dimethyl-3-nitropyridin aus Beispiel 4 in 10 ml Äthanol innerhalb von 10 Minuten zugetropft. Danach wird noch 2 Stunden auf 90 °C erwärmt. Nach dem Abkühlen wird eingeengt, mit Wasser aufgenommen und mit Chloroform ausgeschüttelt. Die Chloroformphase wird mit Natriumsulfat getrocknet und eingeengt. Der Rückstand wird mit äthanolischer Salzsäure ins Hydrochlorid überführt und aus Isopropanol umkristallisiert.

Ausbeute: 1,6 g (52,5% der Theorie), Schmelzpunkt 138–140 °C

$C_{12}$ $H_{20}$ Cl $N_3$ $O_2$ S (MG = 305,83)

Analyse:

Berechnet:

C 47,13 H 6,59 Cl 11,59 N 13,74 S 10,48%

Gefunden:

C 46,68 H 6,54 Cl 11,61 N 13,42 S 10,78%

6. 4-Benzylamino-2,6-dimethyl-3-nitropyridin-hydrochlorid

9,3 g (0,05 Mol) 4-Chlor-2,6-dimethyl-3-nitropyridin und 10,7 g (0,1 Mol) Benzylamin werden in 20 ml Isopropanol 5 Stunden zum Sieden erhitzt. Nach dem Abkühlen wird eingeengt, mit Wasser aufgenommen und mit Chloroform extrahiert. Die Chloroformphase wird mit Natriumsulfat getrocknet und eingeengt. Der Rückstand wird mit äthanolischer Salzsäure ins Hydrochlorid übergeführt und aus Isopropanol umkristallisiert.

Ausbeute: 7,9 g (53,8% der Theorie), Schmelzpunkt 192–193 °C.

$C_{14}$ $H_{16}$ Cl $N_3$ $O_2$ (MG = 293,75)

Analyse

Berechnet:

C 57,24 H 5,49 Cl 12,07 N 14,31%

Gefunden:

C 57,45 H 5,39 Cl 12,18 N 14,12%

Die vorgenannten und die auf analoge Weise hergestellten Verbindungen sind in nachstehender Tabelle 1 zusammengefasst.

Tabelle 1: Verbindung gemäss Formel I

| Beispiel | $R^1$ | $R^2$ | $R^3$ | $R^4$ | Y | isoliert als | Schmelzpunkt °C |
|---|---|---|---|---|---|---|---|
| 1 | 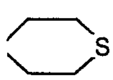 | | $-CH_3$ | $-CH_3$ | $NO_2$ | HCl | 240 (Zers.) |
| 2 | 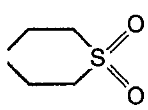 | | $-CH_3$ | $-CH_3$ | $NO_2$ | HCl | 255 (Zers.) |
| 3 | $-CH_2-CH_3$ | $-(CH_2)_2-OH$ | $-CH_3$ | $-CH_3$ | $NO_2$ | HCl | 116–118 |
| 4 | $-CH_2-CH_3$ | $-(CH_2)_2-Cl$ | $-CH_3$ | $-CH_3$ | $NO_2$ | HCl | 175–179 |
| 5 | $-CH_2-CH_3$ | $-(CH_2)_2-S-CH_3$ | $-CH_3$ | $-CH_3$ | $NO_2$ | HCl | 138–140 |
| 6 | H | 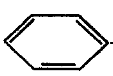$-CH_2-$ | $-CH_3$ | $-CH_3$ | $NO_2$ | HCl | 192–193 |
| 7 | 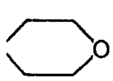 | | $-CH_3$ | $-CH_3$ | $NO_2$ | HCl | 220–223 |

Tabelle 1 (Fortsetzung)

| Beispiel | $R^1$ | $R^2$ | $R^3$ | $R^4$ | Y | isoliert als | Schmelzpunkt °C |
|---|---|---|---|---|---|---|---|
| 8 | | 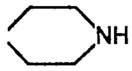 | $-CH_3$ | $-CH_3$ | $NO_2$ | 2 HCl | 260–265 (Zers.) |
| 9 | | 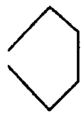 | $-CH_3$ | $-CH_3$ | $NO_2$ | HCl | 240 |
| 10 | | 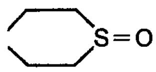 | $-CH_3$ | $-CH_3$ | $NO_2$ | HCl | 240 |
| 11 | | 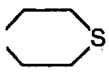 | $-CH_3$ | $-CH_3$ | CN | HCl | 264–267 |
| 12 | | 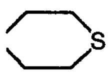 | $-C_3H_7$ | $-C_3H_7$ | $NO_2$ | HCl | 161–163 |
| 13 | | 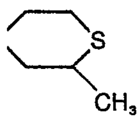 | $-CH_3$ | $-CH_3$ | $-NO_2$ | HCl | 215 (Zers.) |
| 14 | | 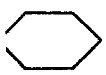 | $-CH_3$ | $-CH_3$ | $-NO_2$ | Base | 69 |
| 15 | H | 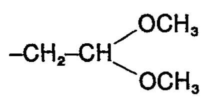 | $-CH_3$ | $-CH_3$ | $NO_2$ | Base | 57 |
| 16 | H | $-CH_2-CH_2-Cl$ | $-CH_3$ | $-CH_3$ | $NO_2$ | HCl | 177 |
| 17 | $-CH_2-CH_3$ | 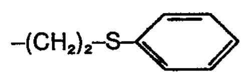 | $-CH_3$ | $-CH_3$ | $NO_2$ | HCl | 71–73 |
| 18 | H | 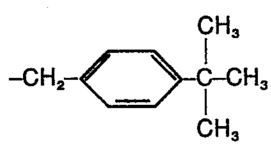 | $-CH_3$ | $-CH_3$ | $NO_2$ | HCl | 202–204 |
| 19 | H | 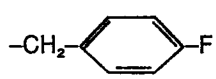 | $-CH_3$ | $-CH_3$ | $NO_2$ | HCl | 210–214 |
| 20 | H | 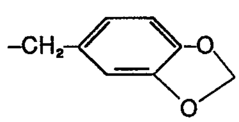 | $-CH_3$ | $-CH_3$ | $NO_2$ | HCl | 188–190 |
| 21 | H | 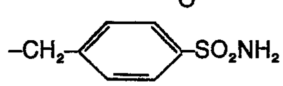 | $-CH_3$ | $-CH_3$ | $NO_2$ | HCl | 222–225 |
| 22 | H | 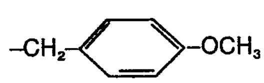 | $-CH_3$ | $-CH_3$ | $NO_2$ | HCl | 159–161 |
| 23 | H | 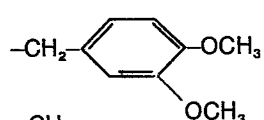 | $-CH_3$ | $-CH_3$ | $NO_2$ | HCl | 222–225 |
| 24 | H | 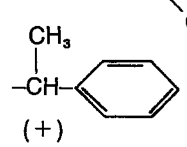 (+) | $-CH_3$ | $-CH_3$ | $NO_2$ | HCl | 194–196 |

Tabelle 1 (Fortsetzung)

| Beispiel | $R^1$ | $R^2$ | $R^3$ | $R^4$ | Y | isoliert als | Schmelzpunkt °C |
|---|---|---|---|---|---|---|---|
| 25 | H | 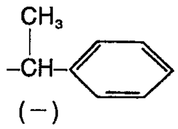 | $-CH_3$ | $-CH_3$ | $NO_2$ | HCl | 194–196 |
| 26 | H | 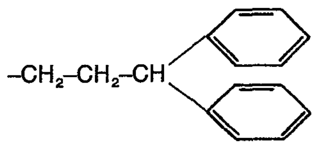 | $-CH_3$ | $-CH_3$ | $NO_2$ | Base | 102–103 |
| 27 | H | 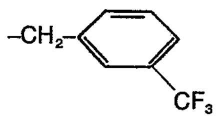 | $-CH_3$ | $-CH_3$ | $NO_2$ | Base | 117–119 |
| 28 | 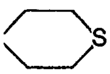 | | $-C_6H_{13}$ | $-CH_3$ | $NO_2$ | HCl | 202–204 (Zers.) |
| 29 | 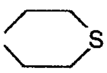 | | H | $-CH_3$ | $NO_2$ | HCl | 213 (Zers.) |
| 30 | H | $-CH_2-CH_2-F$ | $-CH_3$ | $-CH_3$ | $NO_2$ | HCl | 176 |
| 31 | H | 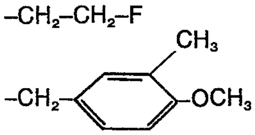 | $-CH_3$ | $-CH_3$ | $NO_2$ | Base | 107–109 |
| 32 | H | $-CH_2-CH_2-Cl$ | $-CH_3$ | $-CH_3$ | CN | HCl | 200–202 |
| 33 | H | $-CH_2-CH_2-CH_2-Cl$ | $-CH_3$ | $-CH_3$ | $NO_2$ | HCl | 150–152 |
| 34 | H | 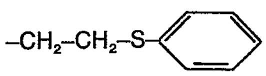 | $-CH_3$ | $-CH_3$ | $NO_2$ | HCl | 102–103 |
| 35 | H | 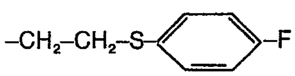 | $-CH_3$ | $-CH_3$ | $NO_2$ | HCl | 153–154 |
| 36 | H | 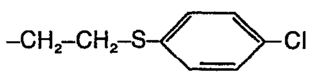 | $-CH_3$ | $-CH_3$ | $NO_2$ | HCl | 113–114 |
| 37 | H | 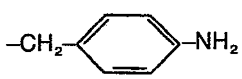 | $-CH_3$ | $-CH_3$ | $NO_2$ | HCl | 142–144 |
| 38 | H | 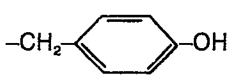 | $-CH_3$ | $-CH_3$ | $NO_2$ | Base | 170–172 |
| 39 | H | 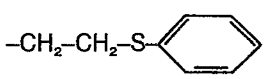 | $-CH_3$ | $-CH_3$ | CN | HCl | 161–162 |
| 40 | H | 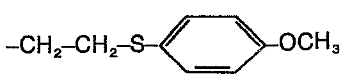 | $-CH_3$ | $-CH_3$ | $NO_2$ | HCl | 159–160 |
| 41 | H | 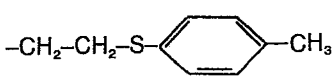 | $-CH_3$ | $-CH_3$ | $NO_2$ | HCl | 194–195 |
| 42 | H | $-CH_2-CH_2-S-CH_3$ | $-CH_3$ | $-CH_3$ | $NO_2$ | HCl | 103–104 |
| 43 | H | 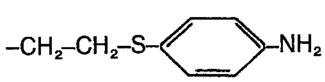 | $-CH_3$ | $-CH_3$ | $NO_2$ | 2 HCl | 246–248 |

Pharmakologische Prüfung und Ergebnisse

1. Bronchospasmolytische Wirkung

Die Prüfung der erfindungsgemässen Verbindungen auf bronchospasmolytische Aktivität erfolgt im wesentlichen mit der von H. Konzett und R. Rössler beschriebenen Versuchsanordnung [Arch. exp. Path. u. Pharmak. 195 (1940) 71] im Vergleich mit dem bekannten Bronchospasmolytikum Theophyllin-Äthylendiamin, bei der die Hemmung experimenteller Bronchialspasmen -ausgelöst durch intravenöse Gabe spasmogener Amine, wie Acetylcholin, Histamin und Serotonin – an Meerschweinchen beiderlei Geschlechts in Urethan-Narkose (1,25 g/kg i.p.) untersucht wird.

Die Prüfsubstanzen wurden in wässriger Lösung entweder intravenös (i.v.) oder intraduodenal (i.d.) verabreicht. Wasserunlösliche Verbindungen wurden in Carboxymethylcellulose-Suspensionen i.d. appliziert. Die $ED_{50}$-Werte, die jene Dosis in mg/kg bezeichnen, bei der der experimentell erzeugte Spasmus auf die Hälfte gegenüber jenem unbehandelter Tiere herabgesetzt ist, wurden graphisch aus den Dosiswirkungskurven bestimmt.

2. Akute Toxizität

Die Bestimmung der $LD_{50}$-Werte bzw. $LD_{50}$-Bereiche erfolgte standardgemäss über die innerhalb von 7 Tagen bei NMRI-Mäusen nach einmaliger intraperitonealer (i.p.) Gabe auftretende Mortalität.

Die Ergebnisse dieser Untersuchungen, die die Überlegenheit der erfindungsgemässen Verbindungen gemäss Formel I gegenüber dem Standardpräparat Theophyllin-Äthylendiamin demonstrieren (insbesondere auch unter Berücksichtigung des günstigeren Verhältnisses von $LD_{50}$ zu $ED_{50}$), sind in der nachfolgenden Tabelle 2 zusammengefasst.

Tabelle 2

| Verbindung des Beispiels | Applikationsart | Bronchospasmolytische Wirkung ($ED_{50}$ in mg/kg) gegenüber Acetylcholin | Histamin | Serotonin | Toxizität $LD_{50}$ (Maus i.p.) mg/kg |
|---|---|---|---|---|---|
| 1 | i.v. | 1,47 | 1,05 | 1,32 | 160* |
| | i.d. | 4,13 | 2,6 | 2,52 | (145–177) |
| 7 | i.v. | 3–10 | 1–3 | | 300–1000 |
| 11 | i.v. | 3 | 1–3 | 3 | 150–300 |
| | i.d. | 3–10 | 1–3 | | |
| 12 | i.v. | 1–3 | 3 | 3–10 | 300–600 |
| 13 | i.v. | 3–10 | 3–10 | | 150–300 |
| 15 | i.v. | 3–10 | >10 | | 400–800 |
| 16 | i.v. | 1–3 | 3 | 1–3 | 150–300 |
| 20 | i.v. | 3–10 | 3 | | |
| 21 | i.v. | 1–3 | 3–10 | | 600–1200 |
| 22 | i.v. | 1–3 | 0,3–1,0 | 1–3 | >600 |
| 30 | i.v. | 3–10 | 1 | 1–3 | 150–300 |
| Theophyllin-Äthylendiamin | i.v. | 8,3 | 5,6 | 7,3 | 217* |
| | i.d. | 10–20 | | | (210–223) |

* bestimmt nach Litchfield und Wilcoxon (J. Pharmacol. exp. Ther. 96 [1949] 99)

**Patentansprüche**

1. Mehrfach substituierte 4-Aminopyridine der Formel

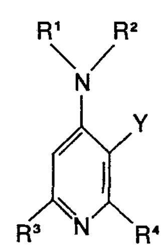

(I)

in der

$R^1$ Wasserstoff oder Alkyl mit bis zu 6 C-Atomen und

$R^2$ a) Alkyl mit bis zu 6 C-Atomen, das bis zu dreifach mit wenigstens einem Rest aus der Gruppe Halogen, Hydroxyl, mit einem Alkohol mit bis zu 3 C-Atomen acetalisiertes Formyl, Alkylthio mit bis zu 3 C-Atomen, Phenylthio, wobei der Phenylring bis zu drei Substituenten der Gruppe Alkyl, Alkoxy, Halogen, Amino und Hydroxy tragen kann, substituiert sein kann, oder

b) Phenylalkyl oder Diphenylalkyl mit bis zu 6 C-Atomen im geraden oder verzweigten Alkylteil, wobei die Phenylringe bis zu dreifach mit wenigstens einem Rest aus der Gruppe Alkyl, Alkoxy, Halogenalkyl mit jeweils bis zu 4 C-Atomen, Halogen, Amino, Hydroxy, der Sulfamoylgruppe und dem Methylendioxyrest substituiert sein könne, bedeuten oder

$R^1$ und $R^2$ zusammen mit dem 4-ständigen N-Atom einen fünf- oder sechsgliedrigen heterocyclischen Ring mit bis zu zwei Heteroatomen bilden, der mit Alkyl bis zu 2 C-Atomen substituiert sein kann, wobei das zweite Heteroatom Sauerstoff, Schwefel, der bis zu zwei Sauerstoffatome tragen kann, oder Stickstoff ist, und

$R^3$ und $R^4$ jeweils gleich oder verschieden und Alkyl mit bis zu 9 C-Atomen, wobei einer der beiden Reste auch Wasserstoff sein kann und

Y eine Nitro- oder Cyanogruppe bedeuten, sowie die physiologisch verträglichen Salze dieser Verbindungen.

2. Verbindungen nach Anspruch 1, gekennzeichnet durch wenigstens eines der Merkmale, dass a) in $R^2$ der Alkylteil des Phenylalkyl- bzw. Diphenylalkylrestes bis zu 4 C-Atome umfasst, b) das Alkyl von $R^3$ und $R^4$ jeweils bis zu 7, vorzugsweise bis zu 4 C-Atome besitzt und c) ein aus $R^1$ und $R^2$ mit dem 4-ständigen N-Atom gebildeter heterocyclischer Ring gesättigt ist.

3. Verbindungen nach Anspruch 1, dadurch gekennzeichnet, dass in Formel I

$R^1$ und $R^2$ zusammen mit dem 4-ständigen N-Atom einen Morpholin- oder Thiomorpholinring bilden und

$R^3$ und $R^4$ jeweils alkyl mit bis zu 4 C-Atomen, und

Y eine Nitro- oder Cyanogruppe

bedeuten.

4. Verbindungen nach Anspruch 1, dadurch gekennzeichnet, dass in Formel I

$R^1$ Wasserstoff oder Alkyl mit bis zu 4 C-Atomen,

$R^2$ mit Halogen, Alkylthio mit bis zu 3 C-Atomen oder einer gegebenenfalls substituierten Phenylthiogruppe substituiertes Alkyl mit bis zu 4 C-Atomen

$R^3$ und $R^4$ jeweils Alkyl mit bis zu 4 C-Atomen und

Y eine Nitro- oder Cyanogruppe

bedeuten.

5. Verbindungen nach Anspruch 1, dadurch gekennzeichnet, dass in Formel I

$R^1$ Wasserstoff

$R^2$ Benzyl, dessen $CH_2$-Gruppe mit Methyl und dessen Phenylring mit Alkoxy mit bis zu 2 C-Atomen, Halogen, der Sulfamoylgruppe oder dem Methylendioxy-Rest substituiert sein kann,

$R^3$ und $R^4$ jeweils Alkyl mit bis zu 4 C-Atomen und

Y eine Nitrogruppe

bedeuten.

6. Verfahren zur Herstellung von 4-Aminopyridinen der Formel I von Anspruch 1 sowie ihrer physiologisch verträglichen Salze, dadurch gekennzeichnet, dass man

a) eine Verbindung der allgemeinen Formel (II)

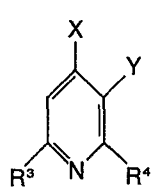

(II)

mit einem Amin der allgemeinen Formel (III)

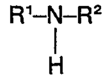

(III)

umsetzt, wobei $R^1$, $R^2$, $R^3$, $R^4$ und Y die im Anspruch 1 angegebenen Bedeutungen haben und X ein Halogenatom darstellt, und das erhaltene Produkt isoliert, oder

b) zur Herstellung von Verbindungen der Formel I, in denen $R^2$ ein Halogenatom enthält, eine entsprechende Verbindung der Formel I, in der $R^2$ eine an Alkyl gebundene Hydroxylgruppe trägt, halogeniert, oder

c) zur Herstellung von Verbindungen der Formel I, in denen $R^2$ eine Alkylthio- oder Phenylthiogruppe enthält, die nach Verfahren (b) erhaltene Halogenverbindung mit einem Mercaptan der Formel IV

$$H{-}S{-}R^5 \qquad (IV)$$

umsetzt, wobei $R^5$ Alkyl mit bis zu 3 Kohlenstoffatomen oder Phenyl bedeutet, das mit den bei $R^2$ (a) genannten Resten substituiert sein kann, oder

d) die nach Verfahren (a) erhaltenen heterocyclischen Thioverbindungen zu Sulfoxyden oder Sulfonen oxidiert,

wobei man die Verbindungen der Formel I entweder als freie Basen isoliert oder mit geeigneten Säuren physiologisch verträgliche Säureadditionssalze bildet.

7. Verfahren nach Anspruch 6, dadurch gekennzeichnet, dass man bei der Umsetzung von Verbindungen der Formel II mit den Aminen der Formel III in einem Lösungs- oder Verteilungsmittel bei Temperaturen zwischen 0 °C und dem Siedepunkt des jeweiligen Lösungsmittels, vorzugsweise zwischen 20°C und 100 °C, arbeitet.

8. Verfahren nach Anspruch 6, dadurch gekennzeichnet, dass man die Halogenierung des Hydroxyalkylgruppen enthaltenden Verbindungen bei 10 bis 100, vorzugsweise 20 bis 70 °C, die Umsetzung mit den Mercaptanen (IV bei 0 bis 150, vorzugsweise 50 bis 100 °C und die Oxydation zu den Sulfoxyden oder Sulfonen bei −20 bis 100, vorzugsweise 0 bis 70 °C durchführt.

9. Verfahren nach Anspruch 6 oder 8, dadurch gekennzeichnet, dass man die Halogenierung in dem jeweiligen Halogenierungsmittel, die Umsetzung mit den Mercaptanen in einem Lösungs- oder Verdünnungsmittel in Gegenwart eines säurebindenden Mittels und die Oxydation zu den Sulfoxyden oder Sulfonen in Wasser als Lösungsmittel durchführt.

10. Arzneimittel, gekennzeichnet durch einen Gehalt an mindestens einer Verbindung der Formel I und/oder einem ihrer Salze nach Ansprüche 1 bis 5.

**Claims**

1. 4-Aminopyridines carrying several substituents and corresponding to the formula

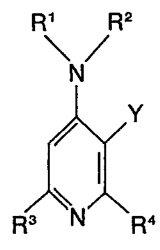

(I)

in which

$R^1$ is hydrogen or alkyl of up to 6 carbon atoms and

$R^2$ is a) alkyl of up to 6 carbon atoms, which may be substituted up to three times with at least one radical from the group of halogen, hydroxy, formyl acetalized with an alcohol of up to 3 carbon atoms, alkylthio of up to 3 carbon atoms, phenylthio, the phenyl ring optionally carrying up to three substituents from the group of alkyl, alkoxy, halogen, amino and hydroxy, or

b) phenylalkyl or diphenyl-alkyl of up to 6 carbon atoms in the linear or branched alkyl moiety, the phenyl rings optionally being substituted up to three times with at least one radical from the group of alkyl, alkoxy, alkyl halide with up to 4 carbon atoms each, halogen, amino, hydroxy, the sulfamoyl group and the methylene dioxy radical, or

$R^1$ and $R^2$, together with the nitrogen atom present in the 4-position, form a five- or six-membered heterocyclic ring optionally substituted with alkyl of up to 2 carbon atoms and including up to two hetero-atoms, the second hetero-atom being oxygen, sulfur optionally carrying up to two oxygen atoms, or nitrogen, and

$R^3$ and $R^4$ which may be identical or different, each are alkyl of up to 9 carbon atoms, one of the two radicals optionally also being hydrogen and

Y is a nitro or a cyano group and the physiologically acceptable salts of these compounds.

2. Compounds as claimed in claim 1 characterised by at least one of the following features, i.e.

(a) in $R^2$ the alkyl moiety of the phenylalkyl or the diphenylalkyl radical includes up to 4 carbon atoms,

(b) the alkyls of $R^3$ and $R^4$ each have up to 7, preferably up to 4 carbon atoms, and

(c) a heterocyclic ring having been formed of $R^1$ and $R^2$ with the N-atom in the 4-position is saturated.

3. Compounds as claimed in claim 1, characterised in that in formula I

$R^1$ and $R^2$ together with the N-atom in the 4-position form a morpholine or thiomorpholine ring, and

$R^3$ and $R^4$ each are alkyl of up to 4 carbon atoms and

Y is a nitro or cyano group.

4. Compounds as claimed in claim 1, characterised in that in formula I

$R^1$ is hydrogen or alkyl of up to 4 carbon atoms,

$R^2$ is alkyl of up to 4 carbon atoms substituted by halogen, alkylthio of up to 3 carbon atoms or by an optionally substituted phenylthio group,

$R^3$ and $R^4$ each are alkyl of up to 4 carbon atoms and

Y is a nitro or cyano group.

5. Compounds as claimed in claim 1, characterised in that in formula I

$R^1$ is hydrogen,

$R^2$ is benzyl, the $CH_2$ group of which may be substituted by methyl and the phenyl ring of which may be substituted by alkoxy of up to 2 carbon atoms, halogen, the sulfamoyl group or the methylenedioxy radical,

$R^3$ and $R^4$ each are alkyl of up to 4 carbon atoms and

Y is a nitro group.

6. Process for the preparation of 4-aminopyridines of the formula I of claim 1, characterised by

(a) reacting a compound of the formula II

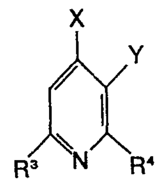

(II)

with an amine of the formula III

$$R^1-\underset{\displaystyle H}{\underset{|}{N}}-R^2 \qquad (III)$$

in which $R^1$, $R^2$, $R^3$, $R^4$ and Y are defined as in claim 1 and X is halogen and isolating the product obtained, or

b) for preparing compounds of the formula I, in which $R^2$ contains a halogen atom, halogenating a corresponding compound of the formula I, in which $R^2$ carries a hydroxyl group bound to alkyl, or

c) for preparing compounds of the formula I, in which $R^2$ contains an alkylthio or phenylthio group, reacting the halogen compound obtained according to process (b) with a mercaptan of the formula IV

$$H-S-R^5 \qquad (IV),$$

in which $R^5$ represents alkyl of up to 3 carbon atoms or phenyl optionally being substituted by the radicals mentioned under $R^2$ (a), or

d) oxidizing the heterocyclic thio compounds obtained from process (a) above to give sulfoxides or sulfones,

and either isolating the compounds of formula I in the form of free bases or forming physiologically acceptable acid addition salts with suitable acids.

7. Process as claimed in claim 6, characterized by carrying out the reaction of compounds of formula II with the amines of formula III in a solvent or dispersing agent at a temperature of from 0 °C to the boiling point of the respective solvent, preferably at a temperature of from 20 to 100 °C.

8. Process as claimed in claim 6, characterized by effecting the halogenation of the compounds containing hydroxyalkyl groups at 10 to 100, preferably from 20 to 70 °C, the reaction with the mercaptans (IV) at 0 to 150, preferably from 50 to 100 °C, and the oxidation to give the sulfoxides or sulfones at −20 to 100, preferably from 0 to 70 °C.

9. Process as claimed in claim 6 or 8, characterized by carrying out the halogenation in the respective halogenating agent, the reaction

with the mercaptans in a solvent or diluent in the presence of an acidbinding agent, and the oxidation to give the sulfoxides or sulfones in water as solvent.

10. Medicament, characterized by a content of at least one compound of formula I and/or one of its salts as claimed in claims 1 to 5.

**Revendications**

1. 4-aminopyridines plusieurs fois substituées formule

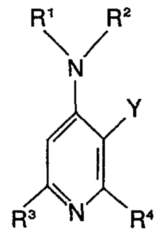

(I)

dans laquelle

$R^1$ représente l'hydrogène ou un groupe alkyle ayant jusqu'à 6 atomes de carbone, et

$R^2$ représente: a) un groupe alkyle ayant jusqu'à 6 atomes de carbone, qui peut être substituè jusqu'à 3 fois avec au moins un radical choisi parmi un halogène ou un groupe hydroxyle, formyle acétalisé avec un alcool ayant jusqu'à 3 atomes de carbone, alkylthio ayant jusqu'à 3 atomes de carbone, ou phénylthio, le noyau phényle pouvant porter jusqu'à trois substituants choisis parmi les groupes alkyle, alcoxy, halogéno, amino er hydroxy; ou

b) un groupe phénylalkyle ou diphénylalkyle ayant jusqu'à 6 atomes de carbone dans la partie alkyle à chaîne droite ou ramifiée, les noyaux phényle pouvant être substitués jusqu'à trois fois avec au moins un radical choisi parmi les groupes alkyle, alcoxy, halogénoalkyle ayant jusqu'à 4 atomes de carbone, halogéno, amino, ou hydroxy, le groupe sulfamoyle et le radical méthylènedioxy; ou

$R^1$ et $R^2$ forment ensemble avec l'atome d'azote en position 4 un noyau hétérocyclique à 5 ou 6 chaînons ayant jusqu'à deux hétéroatomes, qui peut être substitué par un groupe alkyle ayant jusqu'à 2 atomes de carbone, le deuxième hétéroatome étant l'oxygène, le soufre qui peut porter jusqu'à deux atomes d'oxygène, ou l'azote; et

$R^3$ et $R^4$ identiques ou différents, représentent un groupe alkyle ayant jusqu'à 9 atomes de carbone, un des deux radicaux pouvant être également l'hydrogène; et

Y représente un groupe nitro ou cyano

et les sels physiologiquement acceptables de ces composés.

2. Composés selon la revendication 1, caractérisés par au moins und des caractéristiques suivantes, à savoir (a) dans $R^2$ la partie alkyle du radical phénylalkyle ou diphénylalkyle comprend jusqu'à 4 atomes de carbone, b) le groupe alkyle de $R^3$ et $R^4$ possède jusqu'à 7, de préférence jusqu'à 4 atomes de carbone et c) un des hétérocycles, formés à partir de $R^1$ et $R^2$ avec l'atome d'azote en position 4, est saturé.

3. Composés selon la revendication 1, caractérisés en ce que dans la formule I:

$R^1$ et $R^2$ forment ensemble avec l'atome d'azote en position 4 un noyau morpholine ou thiomorpholine; et

$R^3$ et $R^4$ représentent chacun un groupe alkyle ayant jusqu'à 4 atomes de carbone, et

Y représente un groupe nitro ou cyano.

4. Composés selon la revendication 1, caractérisés en ce que la formule I:

$R^1$ représente l'hydrogène ou un groupe alkyle ayant jusqu'à 4 atomes de carbone,

$R^2$ représente un groupe alkyle ayant jusqu'à 4 atomes de carbone, substitué par un halogène, un groupe alkylthio ayant jusqu'à 3 atomes de carbone ou un groupe phénylthio éventuellement substitué.

$R^3$ et $R^4$ représentent chacun un groupe alkyle ayant jusqu'à 4 atomes de carbone; et

Y représente un groupe nitro ou cyano.

5. Composés selon la revendication 1, caractérisés en ce que dans la formule I:

$R^1$ représente l'hydrogène,

$R^2$ représente un radical benzyle dont le groupe $CH_2$ peut être substitué avec un groupe méthyle et dont le noyau phényle peut être substitué avec un groupe alcoxy ayant jusqu'à 2 atomes de carbone, un halogène, le groupe sulfamoyle ou le radical méthylènedioxy.

$R^3$ et $R^4$ représentent chacun un groupe alkyle ayant jusqu'à 4 atomes de carbone; et

Y représente un groupe nitro.

6. Procédé de préparation de 4-aminopyridines formule I selon la revendication 1, ainsi que leurs sels physiologiquement acceptables, caractérisé en ce que:

a) on fait réagir un composé de formule générale (II)

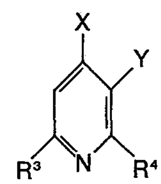

(II)

avec une amine de formule générale (III)

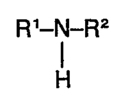

(III)

$R^1$, $R^2$, $R^3$, $R^4$ et Y ayant les significations indiquées dans la revendication 1 et X représentant un atome d'halogène et on isole le produit obtenu, ou

b) pour préparer des composés de formule I dans lesquels $R^2$ contient un atome d'halogène, on halogène un composé correspondant de formule I dans lequel $R^2$ porte un groupe hydroxyle fixé sur un groupe alkyle, ou

c) pour préparer des composés de formule I dans lesquels $R^2$ contient un groupe alkylthio ou phénylthio, on fait réagir le composé halogéné obtenu selon le mode opératoire (b) avec un mercaptan de formule IV

$$H{-}S{-}R^5 \quad (IV)$$

$R^5$ représentant un groupe alkyle ayant jusqu'à 3 atomes de carbone ou un groupe phényle qui peut être substitué avec les radicaux indiqués pour $R^2$ (a), ou

d) on oxyde les composés thio hétérocycliques obtenus selon le mode opératoire (a) en sulfoxydes ou en sulfones, les composés de formule I étant isolés sous forme de bases libres ou, avec des acides appropriés, on forme des sels d'addition avec des acides physiologiquement acceptables.

7. Procédé selon la revendication 6, caractérisé en ce que, pour la réaction des composés de formule II avec les amines de formule III, on opère dans un solvant ou un diluant à des températures comprises entre 0 °C et le point d'ébullition du solvant utilisé, de préférence entre 20 et 100 °C.

8. Procédé selon la revendication 6, caractérisé en ce qu'on effectue l'halogénation des composés contenant des groupes hydroxy-alkyle entre 10 et 100, de préférence entre 20 et 70 °C, la réaction avec les mercaptans (IV) entre 0 et 150, de préférence entre 50 et 100 °C, et l'oxydation en sulfoxydes ou en sulfones entre 20 et 100, de préférence entre 0 et 70 °C.

9. Procédé selon la revendication 6 ou 8, caractérisé en ce qu'on effectue l'halogénation dans l'agent d'halogénation choisi, la réaction avec les mercaptans dans un solvant ou un diluant en présence d'un agent fixant les acides et l'oxydation en sulfoxydes ou en sulfones dans l'eau comme solvant.

10. Médicament caractérisé par une teneur en au moins un composé de formule I et/ou un de ses sels, selon l'une quelconque des revendications 1 à 5.